# EUROPEAN PATENT SPECIFICATION

(11) **EP 2 395 970 B1**
(45) Date of publication and mention of the grant of the patent: **17.04.2013**
(21) Application number: 10704865.4
(22) Date of filing: 05.02.2010
(51) Int. Cl.: A61K 9/00, A61K 31/19

(54) **VALPROIC ACID DRUG DELIVERY SYSTEMS AND INTRAOCULAR THERAPEUTIC USES THEREOF**
SYSTEME ZUR ABGABE VON VALPROINSÄURE-ARZNEIMITTELN UND IHRE INTRAOKULARE THERAPEUTISCHE VERWENDUNGEN
SYSTÈMES D'ADMINISTRATION DE MÉDICAMENT À BASE D'ACIDE VALPROÏQUE ET LEURS UTILISATIONS THÉRAPEUTIQUES INTRAOCULAIRES

(30) Priority: 11.02.2009 US 369705
(43) Date of publication of application: 21.12.2011
(73) Proprietor: Allergan, Inc., Irvine, CA 92612 (US)
(72) Inventor: BACIU, Peter, C., Laguna Nigel, California 92677 (US); HUGHES, Patrick, M., Aliso Viejo, California 92656 (US); BLANDA, Wendy, M., Tustin, California 92780 (US)
(74) Representative: HOFFMANN EITLE
(86) International application number: PCT/US2010/023308
(87) International publication number: WO 2010/093564

(56) References cited:
- WO-A1-2008/038316
- WO-A2-2006/041942
- WO-A2-2008/073295
- US-A- 4 327 725
- US-A- 5 660 851
- US-A1- 2007 260 203
- KUMAR G JANORIA ET AL: "Novel approaches to retinal drug delivery" EXPERT OPINION ON DRUG DELIVERY, ASHLEY PUBLICATIONS, GB, vol. 4, 1 July 2007 (2007-07-01), pages 371-388, XP009131521 ISSN: 1742-5247

## Description

### BACKGROUND

The present invention relates to valproic acid drug delivery systems and therapeutic use of such systems. In particular the present invention relates to an intraocular, valproic acid, sustained release drug delivery system for treatment of retinal diseases and conditions. Valproic acid (2-propylpentanoic acid; C₈H₁₆O₂) and it's various salts (valproates, such as sodium valproate, calcium valproate and valproate semisodium and other valproate alkali and alkali earth salts), derivatives (such as divalproex; 2-n-propyl-3-aminopentanoic acid, and; 2-n-propyl-4-aminopentanoic acid), analogs (such as 2-n-propyl-4-hexynoic acid) and esters have been administered systemically (for example by intravenous and oral routes) to treat epilepsy, bipolar disorder, depression, migraine headaches and schizophrenia. Anti-convulsant effect of valproic acid is believed due to inhibition of voltage-gated sodium channels and T-type calcium channels. Therapeutic mood and pain alleviation may be due to valproic acid activity as a GABA transaminase inhibitor which permits formation of increased levels of GABA neurotransmitter in the brain. Approved therapeutic formulations of valproic acid include Depakene (Abbott Laboratories), Convulex (Pfizer), Stavzor (Noven Pharmaceuticals), and Depakine (Sanofi Aventis). Valproic acid have been found cytotoxic to various cancer cells possibly due to inhibition of histone deacetylase resulting in generation of oxidative radicals in tumor tissue.

Unfortunately, therapeutic use of systemic valproic acid has significant side effects including liver toxicity, bone loss, blockage of fatty acid metabolism and resulting weight gain. Additionally, it has been widely reported that treatment with valproic acid has numerous deleterious effects on vision including deficits in VEP (visual evoked potential), visual field defects and degraded color vision. See eg Tilz C., et al. Visual field defect during therapy with valproic acid, Eur J Neurol, 2007 Aug; 1498): 929-32 (concentric visual field defect); Geller A., et al., Epilepsy and medication effects on the pattern visual evoked potential, Doc Ophthalmol, 2005 Jan; 110(1):121 -31. Furthermore, administration of valproic acid to mammals has been shown to suppress ERG a- and b-wave amplitudes as well as VEP. See eg Goto, Y, et al., The long-term effects of antiepileptic drugs on the visual system in rats: electrophysiological and histopathological studies. Clin Neurophysiol. 1148: 1395-402 (2003). Similarly, and as noted above, in epileptic patients valproic acid therapy negatively affects vision (Verrotti, A., et al., Color vision and macular recovery time in epileptic adolescents treated with valproate and carbamazepine, Eur J Neurol. 137: 736-41 (2006), and; Verrotti, A., et al., Effects of antiepileptic drugs on evoked potentials in epileptic children, Pediatr Neurol. 235: 397-402 (2000)) for example by reducing color vision (Verrotti (2006) *Id* and Sorri, I., et al., Visual function in epilepsy patients treated with initial valproate monotherapy, Seizure. 146: 367-70 (2005); Verrotti, A. et al., Color vision in epileptic adolescents treated with valproate and carbamazepine, Seizure. 136: 411-7 (2004), and; Verrotti, A., et al., Antiepileptic drugs and visual function, Pediatr Neurol. 366: 353-60 (2007)).

Nau et. al., Biopharm Drug Dispos. 1983 Apr-Jun;4(2):173-82, discusses a refillable non-erodible silastic reservoir, subcutaneous implant of valproic acid for systemic drug delivery. Lopez et. al., Material Letters, Volume 60, Issue 23, October 2006; 2903-2908, discusses a non-erodible TiO2-SiO2 xerogel reservoir for intra cerebral administration of valproic acid. Relevant prior art includes US5660581, US2007260203 and US4327725.

It would be advantageous to have a sustained release valproic acid containing drug delivery system (i.e. formulated as valproic acid containing implants, microspheres or as high viscosity valproic acid containing composition) configured for local intraocular (as opposed to topical or systemic) administration to treat a retinal disease or condition, without significant (i.e. clinically observable) vision deficits resulting.

### SUMMARY

The present invention provides a valproic acid drug delivery system for treatment of retinal diseases and conditions. The system is preferably in the form of an implant, microspheres or as high viscosity drug containing composition which provides for extended intraocular release of the valproic acid therapeutic agent. The drug delivery system can release the valproic acid over a relatively long period of time, for example, for at least about one week or for example for between one week and one year, such as over two weeks, one month, two months or over three months or longer, after intraocular (i.e. intrascleral [such as subconjunctival] or intravitreal) administration of the valproic acid containing drug delivery system. Such extended release times facilitate successful treatment result. In addition, administering the drug delivery system to an intraocular location provides both a high, local therapeutic level of the valproic acid at the intraocular (retinal) target tissue and importantly eliminates or substantially eliminates presence of toxic valproic acid intermediates and metabolites at the site of the intraocular target tissue.

An embodiment of our invention is an intraocular drug delivery system for treating a retinal condition comprising a bioerodible polymer, and a valproic acid associated with the bioerodible polymer. The polymer can comprises from about 10% to about 95% by weight of the drug delivery system. The valproic acid comprises from about 10% to about 95% by weight of the drug delivery system. The polymer can be a polylactic polyglycolic acid copolymer (PLGA), a polylactic acid polymer (PLA) and/or a high viscosity hyaluronic acid. The valproic acid is associated with the polymer by being homogenous mixed with the polymer. The drug delivery system of can be an implant, a population of microspheres and/or a solution or suspension of the valproic acid in a high viscosity, polymeric hyaluronic acid.

A detailed embodiment within the scope of our invention is an intraocular drug delivery implant for treating a retinal condition comprising 10 to 30 weight percent sodium valproate and 70 to 90 weight PLGA, the implant formed by homogenously mixing the valproate and the PLGA, the mixture then heated to a temperature between about 40 and about 180 C, followed by extrusion of the implant.

Our invention also encompasses a method for treating a retinal condition by intraocular administration to a patient with a retinal condition of a drug delivery system comprising a bioerodible polymer, and a valproic acid associated with the bioerodible polymer. The drug delivery system can be administered to the vitreous or to an intrascleral location, such as a subtenon location. The drug delivery system can be a sustained release monolithic implant capable of releasing a therapeutic amount of the valproic acid for between about one week and about one year. The retinal condition can be for example macular edema.

Our invention encompasses a method for reducing retinal tissue oxidative stress in a human patient by intravitreal administration to a human patient with oxidative stress retinal cells of a drug delivery system comprising a bioerodible polymer, and a valproic acid associated with the bioerodible polymer. The oxidative stress retinal tissue contains an excess level of a reactive oxygen species selected from the group consisting of peroxinitrate, super oxide, singlet oxygen, hydrogen peroxide, hypochlorite and hydroxy radical. The method enhances the ability of the retina to respond to oxidative stress by either reducing the excess level of reactive oxygen species (and their oxidative adducts) to a normal level of reactive oxygen species or by enhancing the resistance of the retina to oxidative damage, as determined by a process selected from the group consisting of reactive oxygen sensing dyes, high-performance liquid chromatography (HPLC), immunohistochemistry, Western blotting, enzyme-linked-immunosorbent serologic assay (ELISA), tandem mass spectrometry (MS-MS) and presence or upregulation of oxidative stress response gene/protein or transcription factors.

Thus, an embodiment of our invention is a drug delivery system for intraocular use to treat an ocular condition. The system can comprise a plurality of microspheres made of a bioerodible polymer, and a valproic acid therapeutic agent and/or it's salts, esters and derivatives, contained by the microspheres. The microspheres can comprise from about 1 % to about 99% by weight of the polymer and the polymer can be a PLGA and/or PLA. Additionally, the microspheres can have an average greatest dimension in a range of from about 5 microns to about 1 mm, for example the microspheres can have a mean diameter between about 15 microns and about 55 microns and the therapeutic agent can comprise from about 0.1 % to about 90% by weight of the microspheres, such as between about 8 to 15 weight% valproic acid.

In another embodiment of our invention the composition can include a high viscosity hyaluronic acid and the ocular condition treated can be a retinal disease. A detailed embodiment of our invention is a drug delivery system for intraocular use to treat glaucoma comprising a plurality of microspheres made from a PLGA and/or PLA, valproic acid contained by the microspheres, and a high viscosity hyaluronic acid.

Another embodiment of our invention is a drug delivery system for intraocular use to treat a retinal disease comprising a sustained release implant made from a PLGA polymer, a PLA polymer, and a PEG co-solvent, and; valproic acid contained by the implant, wherein the implant comprises about 30 weight percent valproic acid and the implant can release the valproic acid over a period of time of at least 5, 7, 10, 14, 20, 30, 40, 50, 60, 70 or up to 180 days.

Another embodiment of our invention is a method of treating a retinal disease or condition by intraocular administration to a patient with a retinal disease or condition of a drug delivery system comprising the implant or a plurality of microspheres made from a PLGA and/or PLA; a valproic acid contained by the microspheres, and a high viscosity hyaluronic acid (HA), thereby treating the retinal disease or condition. Preferably, the HA is used with the plurality of microspheres formulation but not with a drug delivery system which comprises a single implant to be administered. The microspheres can release the valproic acid for at least about one week after the administration step. The intraocular administration step can be carried out by injection into the sub-tenon space, such as into the anterior sub-tenon space or into the vitreous and the drug delivery system can slow down or reverse progression of a retinal disease or condition, for example by (at the end of the treatment phase when the drug delivery system has by at least 80% bioeroded and released at least 80% of the valproic acid contained by the drug delivery system) increasing macular thickness (eg by 5% to 50%), reducing retinal edema (eg by 5% to 100%), reducing retinal vein occlusion (eg by 5% to 100%) and by maintaining or improving visual acuity (eg improvement of three or more lines in best measured visual acuity [BMVA]).

### DRAWINGS

Aspects of the present invention are illustrated by the following drawing.

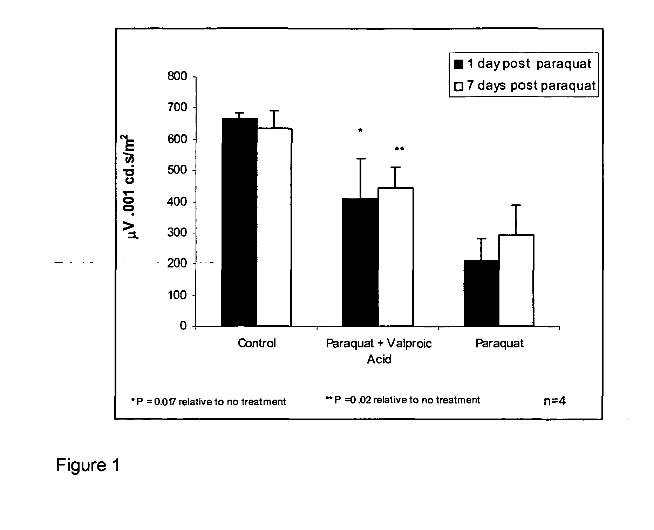

Figure 1 is a bar graph showing Example 2 results. The Y axis is a electroretinogram (ERG) measurement in microvolts .001 cd.s/m², where cd.s/ m² is candela per square meter, a measure of the light flash intensity used, and the X axis shows results (ERG) for the three mice groups studied, each group tested at one and at seven days post paraquat induced retinal oxidative stress.

### DESCRIPTION

Our invention is based on the discovery that a valproic acid containing drug delivery system administered intraocular can treat a retinal disease or condition. With the present valproic acid containing drug delivery system (implant, microspheres or high viscosity carrier) the amount of the valproic acid released into the eye for a period of time greater than about five days after the drug delivery system is placed in the eye is effective in treating or reducing a symptom of a retinal disease or condition, such as by increasing macular thickness, reducing retinal edema, reducing retinal vein occlusion, and/or by maintaining or improving visual acuity and color vision. We determined that systemic valproic acid induced retinal deficits are apparently be due to formation of toxic valproic metabolites. Hence a local (intraocular) administration of valproic acid can prevent presentation of most if not all such toxic byproducts at a retinal target tissue. We then determined through experiment that locally delivered valproic acid can have a beneficial therapeutic effect upon a retinal disease or condition. In pursuit of this therapy we made valproic acid containing drug delivery systems intended for intraocular administration.

Our invention encompasses controlled or sustained delivery of valproic and and/or its salts for the treatment of retinal diseases by direct intraocular implantation of a polymeric drug delivery system containing valproic acid and/or its salts. The drug delivery system can include other active agents and excipients. Valproic acid can be released from the drug delivery system by diffusion, erosion, dissolution or osmosis. The valproic acid can be released from the drug delivery system over a period of about one week, ten days, fourteen days, thirty days, sixty days or up to one year. The polymeric component of the drug delivery system can comprise a bioerodible or non-erodible polymer or polymers. Useful bioerodible polymers include poly-lactide-co-glycolide (PLGA and PLA), polyesters, poly (ortho ester), poly(phosphazine), poly (phosphate ester), polycaprolactone, natural polymers such as gelatin or collagen, or a polymeric blends. The drug delivery system can be a solid implant (monolithic, in which the valproic acid is homogenously distributed), semisolid or viscoelastic. Administration of the drug delivery system can be accomplished via intravitreal injection or implantation.

As set forth in more detail in the Examples *supra* our valproic acid drug delivery system invention is based upon the discoveries that: (1) even though small molecules such as valproic acid are eliminated from the eye extremely rapidly with half-lives of a few hours it is theoretically feasible to deliver valproic acid to intraocular tissues at therapeutic levels over a period of eg one week, or for a period of time between 2 months and to a year; (2) systemic valproic acid causes negative vision effects; (3) the negative vision effects of systemic valproic acid are probably due to valproic acid metabolites generated by hepatic metabolism; (4) counter intuitively in light of the fact that valproic acid is known to cause oxidative stress in tissues (and is being investigated to use that property to the detriment of tumor cells), valproic acid can be used protects against oxidative stress in retinal tissues; (5) a method for the intraocular delivery of valproic acid and its salts for the treatment of intraocular diseases is feasible; (6) a method to reduce the intraocular toxicity of locally delivered valproic acid is feasible; (7) compositions of bioerodible polymeric delivery systems and valproic acid for the treatment of retinal diseases can be prepared, and; (8) compositions of bioerodible polymeric delivery systems and valproic acid with reduced local toxicity can be prepared.

Delivery of drugs to the optic nerve, retina, vitreous and uveal tract is typically achieved by high systemic dosing which can cause toxicity or toxic metabolites, intra-ocular injections or other heroic measures. Penetration of systemically administered drugs into the retina is severely restricted by the blood-retinal barriers (BRB) for most compounds. We determined that local delivery of valproic acid (in an intraocular drug delivery system) can prevent systemic toxicities and mitigate the BRB.

### Definitions

The following definitions are used herein.

"About" means plus or minus ten percent of the number, parameter or characteristic so qualified.

"Biodegradable polymer" means a polymer or polymers which degrade in vivo, and wherein erosion of the polymer or polymers over time occurs concurrent with or subsequent to release of the therapeutic agent. The terms "biodegradable" and "bioerodible" are equivalent and are used interchangeably herein. A biodegradable polymer may be a homopolymer, a copolymer, or a polymer comprising more than two different polymeric units. The polymer can be a gel or hydrogel type polymer, PLA or PLGA polymer or mixtures or derivatives thereof.

"Microsphere" and "microparticle" are used synonymously to refer to a small diameter or dimension (see below) device or element that is structured, sized, or otherwise configured to be administered subconjunctivally (i.e. sub-tenon) or into the vitreous. Microspheres or microparticles includes particles, micro or nanospheres, small fragments, microparticles, nanoparticles, fine powders and the like comprising a biocompatible matrix encapsulating or incorporating a therapeutic agent. Microspheres are generally biocompatible with physiological conditions of an eye and do not cause significant adverse side effects. Microspheres administered intraocular can be used safely without disrupting vision of the eye. Microspheres have a maximum dimension, such as diameter or length, less than 1 mm. For example, microparticles can have a maximum dimension less than about 500 µm. Microspheres can also have a maximum dimension no greater than about 200 µm, or may have a maximum dimension from about 30 µm to about 50 µm, among other sizes. An "implant" is a drug delivery device which is considerably larger than a microsphere, and whereas a plurality (i.e. hundreds or thousands)) of microspheres are administered to treat an ocular condition (such as glaucoma) usually only one to at most six implants are administered for the same purpose.

"Ocular region" or "ocular site" means any area of the eyeball, including the anterior and posterior segment of the eye, and which generally includes, but is not limited to, any functional (e.g., for vision) or structural tissues found in the eyeball, or tissues or cellular layers that partly or completely line the interior or exterior of the eyeball. Specific examples of areas of the eyeball in an ocular region include the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

"Ocular condition" means a disease, ailment or condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball.

An anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves the conjunctiva, the cornea, the anterior chamber, the iris, the posterior chamber (behind the retina but in front of the posterior wall of the lens capsule), the lens or the lens capsule and blood vessels and nerve which vascularize or innervate an anterior ocular region or site.

Thus, an anterior ocular condition can include a disease, ailment or condition, such as for example, aphakia; pseudophakia; astigmatism; blepharospasm; cataract; conjunctival diseases; conjunctivitis; corneal diseases;, corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

A posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site.

Thus, a posterior ocular condition can include a disease, ailment or condition, such as for example, acute macular neuroretinopathy; Behcet's disease; choroidal neovascularization; diabetic uveitis; histoplasmosis; infections, such as fungal or viral-caused infections; macular degeneration, such as acute macular degeneration, non-exudative age related macular degeneration and exudative age related macular degeneration; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial occlusive disease, retinal detachment, uveitic retinal disease; sympathetic opthalmia; Vogt Koyanagi-Harada (VKH) syndrome; uveal diffusion; a posterior ocular condition caused by or influenced by an ocular laser treatment; posterior ocular conditions caused by or influenced by a photodynamic therapy, photocoagulation, radiation retinopathy, epiretinal membrane disorders, branch retinal vein occlusion, anterior ischemic optic neuropathy, non-retinopathy diabetic retinal dysfunction, retinitis pigmentosa, and glaucoma. Glaucoma can be considered a posterior ocular condition because the therapeutic goal is to prevent the loss of or reduce the occurrence of loss of vision due to damage to or loss of retinal cells or optic nerve cells (i.e. neuroprotection).

"Oxidative stress" with regard to a retinal tissue means the condition which exists when the production of one or more reactive oxygen species (for example peroxinitrate, super oxide, singlet oxygen, hydrogen peroxide, hypochlorite and/or or hydroxy radical) and/or oxidative adducts of retinal tissue protein, lipid or DNA (for example nitrotyrosine, acrolein and/ or 8-OHdG) exceeds the ability of the retinal tissue to reduce the reactive oxygen species and/or oxidative adducts to a level which does not cause alterations in cellular/tissue function (for example by causing oxidative damage to retinal tissue lipids, proteins and/or DNA). The former ("exceeds") being referred to as an excess level of reactive oxygen species and the latter ("does not cause") to a normal level of clinically relevant retinal function (due for example to presence in retinal tissue of a normal level of reactive oxygen species). Presence of retinal tissue oxidative stress can be determined by any number of known methods including but not limited to reactive oxygen sensing dyes, high-performance liquid chromatography (HPLC), immunohistochemistry, Western blotting, enzyme-linked-immunosorbent serologic assay (ELISA) and tandem mass spectrometry (MS-MS). Additionally retinal tissue oxidative stress can be determined from the presence or upregulation of oxidative stress response gene/protein or transcription factors.

"Therapeutically effective amount" means level or amount of agent needed to treat an ocular condition, or reduce or prevent ocular injury or damage without causing significant negative or adverse side effects to the eye or a region of the eye. In view of the above, a therapeutically effective amount of a therapeutic agent, such as a valproic acid, is an amount that is effective in reducing at least one symptom of an ocular condition.

"Valproic acid and "valproate" are used herein synonymously and include valproic acid (2-propylpentanoic acid), salts of valproic acid (valproates, such as sodium valproate, calcium valproate and valproate semisodium and other valproate alkali and alkali earth salts), derivatives of valproic acid (such as divalproex; 2-n-propyl-3-aminopentanoic acid, and; 2-n-propyl-4-aminopentanoic acid) and valproic acid analogs (such as 2-n-propyl-4-hexynoic acid) and esters of valproic acid.

We have developed implants and microspheres which can release drug loads over various time periods. These implants or microspheres, which when inserted into the subconjunctival (such as a sub-tenon) space or into the vitreous of an eye provide therapeutic levels of a valproic acid, for extended periods of time (e.g., for about one week or more). The disclosed implants and microspheres are effective in treating ocular conditions, such as ocular conditions associated with a retinal disease or condition, such as macula edema, macular degeneration, retinal neovascularization and retinal vein occlusion.

Additionally, we have developed novel methods for making implants and microspheres. The valproic acid of the present implants and microspheres is preferably from about 1 % to 90% by weight of the microspheres. More preferably, the valproic acid is from about 5% to about 30% by weight of the implant or microspheres. In a preferred embodiment, the valproic acid comprises about 10% by weight of the microsphere (e.g., 5%-15%). In another embodiment, the valproic acid comprises about 40% by weight of the microspheres.

Suitable polymeric materials or compositions for use in the implant or microspheres include those materials which are compatible, that is biocompatible, with the eye so as to cause no substantial interference with the functioning or physiology of the eye. Such materials preferably are at least partially and more preferably substantially completely biodegradable or bioerodible.

Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than about 5%, or less than about 1 % of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, FL 1987, pp 39-90, which describes encapsulation for controlled drug delivery, may find use in the present microspheres.

Of additional interest are polymers of hydroxyaliphatic carboxylic acids, either homopolymers or copolymers, and polysaccharides. Polyesters of interest include polymers of D-lactic acid, L-lactic acid, racemic lactic acid, glycolic acid, polycaprolactone, and combinations thereof. Generally, by employing the L-lactate or D-lactate, a slowly eroding polymer or polymeric material is achieved, while erosion is substantially enhanced with the lactate racemate.

Among the useful polysaccharides are, without limitation, calcium alginate, and functionalized celluloses, particularly carboxymethylcellulose esters characterized by being water insoluble, a molecular weight of about 5 kD to 500 kD, for example.

Other polymers of interest include, without limitation, polyvinyl alcohol, polyesters, polyethers and combinations thereof which are biocompatible and may be biodegradable and/or bioerodible.

Some preferred characteristics of the polymers or polymeric materials for use in the present invention may include biocompatibility, compatibility with the selected therapeutic agent, ease of use of the polymer in making the drug delivery systems of the present invention, a half-life in the physiological environment of at least about 6 hours, preferably greater than about one day, and water insolubility.

The biodegradable polymeric materials which are included to form the matrix are desirably subject to enzymatic or hydrolytic instability. Water soluble polymers may be cross-linked with hydrolytic or biodegradable unstable crosslinks to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer and hence the extended release profile of the implant is the relative average molecular weight of the polymeric composition employed in the microspheres. Different molecular weights of the same or different polymeric compositions may be included in the microspheres to modulate the release profile. For valproic acid implants, the relative average molecular weight of the polymer will preferably range from about 4 to about 25 kD, more preferably from about 5 to about 20 kD, and most preferably from about 5 to about 15 kD.

In some implants and microspheres, copolymers of glycolic acid and lactic acid are used, where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the microspheres. The percentage of polylactic acid in the polylactic acid polyglycolic acid (PLGA) copolymer can be 0-100%, preferably about 15-85%, more preferably about 35-65%. In some implants, a 50/50 PLGA copolymer is used.

The biodegradable polymer matrix of the subconjunctival and/or intravitreal implants and microspheres may comprise a mixture of two or more biodegradable polymers. For example, the implants and microspheres may comprise a mixture of a first biodegradable polymer and a different second biodegradable polymer. One or more of the biodegradable polymers may have terminal acid groups.

Release of a drug from an erodible polymer is the consequence of several mechanisms or combinations of mechanisms. Some of these mechanisms include desorption from the implant or microsphere's surface, dissolution, diffusion through porous channels of the hydrated polymer and erosion. Erosion can be bulk or surface or a combination of both. As discussed herein, the matrix of the microspheres may release drug at a rate effective to sustain release of an amount of the valproic acid for more than one week after implantation into an eye. In certain microspheres, therapeutic amounts of valproic acid are released for no more than about 3-30 days after administration to the subconjunctival space. For example, a microsphere may comprise valproic acid, and the matrix of the microsphere degrades at a rate effective to sustain release of a therapeutically effective amount of valproic acid for about one month after being placed under the conjunctiva. As another example, the microspheres may comprise valproic acid, and the matrix releases drug at a rate effective to sustain release of a therapeutically effective amount of valproic acid for more than thirty days, such as for about six months.

One example of the biodegradable implant or microsphere comprises valproic acid associated with a biodegradable polymer matrix, which comprises a mixture of different biodegradable polymers. At least one of the biodegradable polymers is a polylactide having a molecular weight of about 63.3 kD. A second biodegradable polymer is a polylactide having a molecular weight of about 14 kD. Such a mixture is effective in sustaining release of a therapeutically effective amount of the valproic acid for a time period greater than about one month from the time the microspheres are placed administered under the conjuctiva.

Another example of a biodegradable implant or microsphere comprises valproic acid associated with a biodegradable polymer matrix, which comprises a mixture of different biodegradable polymers, each biodegradable polymer having an inherent viscosity from about 0.16 dl/g to about 1.0 dl/g. For example, one of the biodegradable polymers may have an inherent viscosity of about 0.3 dl/g. A second biodegradable polymer may have an inherent viscosity of about 1.0 dl/g. Additional microspheres may comprise biodegradable polymers that have an inherent viscosity between about 0.2 dl/g and 0.5 dl/g. The inherent viscosities identified above may be determined in 0.1 % chloroform at 25°C.

The release of the valproic acid from an implant or microspheres into the vitreous or subconjuctiva may include an initial burst of release followed by a gradual increase in the amount of the valproic acid released, or the release may include an initial delay in release of the valproic acid followed by an increase in release. When the microspheres are substantially completely degraded, the percent of the valproic acid that has been released is about one hundred. The implants and microspheres disclosed herein do not completely release, or release about 100% of the valproic acid, until after one week or more of being placed in an eye.

It may be desirable to provide a relatively constant rate of release of the valproic acid from the microspheres over the life of the implanted or injected microspheres. For example, it may be desirable for the valproic acid to be released in amounts from about 0.01 µg to about 2 µg per day for the life of the implant or microspheres. However, the release rate may change to either increase or decrease depending on the formulation of the biodegradable polymer matrix. In addition, the release profile of the valproic acid may include one or more linear portions and/or one or more non-linear portions. Preferably, the release rate is greater than zero once the implant or microspheres has begun to degrade or erode.

The implants and microspheres can be monolithic, i.e. having the active agent or agents homogenously distributed through the polymeric matrix, or encapsulated, where a reservoir of active agent is encapsulated by the polymeric matrix. Due to ease of manufacture, monolithic implants are usually preferred over encapsulated (reservoir type) forms. However, the greater control afforded by the encapsulated microspheres may be of benefit in some circumstances, where the therapeutic level of the drug falls within a narrow window. In addition, the therapeutic agent (preferably valproic acid) can be distributed in a non-homogenous pattern in the matrix. For example, the microspheres may include a portion that has a greater concentration of the valproic acid relative to a second portion of the microspheres.

The implants and microspheres disclosed herein may have a size of between about 5 µm and about 1 mm, or between about 10 µm and about 0.8 mm for administration with a needle. For needle-injected microspheres, the microsphere may have any appropriate dimensions so long as the longest dimension of the microsphere permits the microsphere to move through a needle. This is generally not a problem in the administration of microspheres. The subconjunctival space in humans is able to accommodate relatively large volumes of microspheres, for example, about 100 µl, or about 150 µl, or about 50-200 µl or more.

The total weight of implant or microsphere in a single dosage an optimal amount, depending on the volume of the subconjunctival space and the activity or solubility of the active agent. Most often, the dose is usually about 0.1 mg to about 200 mg of implant or microspheres per dose. For example, a single subconjunctival injection may contain about 1 mg, 3 mg, or about 5 mg, or about 8 mg, or about 10 mg, or about 100 mg or about 150 mg, or about 175 mg, or about 200 mg of microspheres, including the incorporated therapeutic agent. For non-human individuals, the dimensions and total weight of the implant or microsphere(s) may be larger or smaller, depending on the type of individual.

The dosage of the therapeutic agent (i.e. valproic acid) in the implant or microspheres is generally in the range from about 0.001 % to about 100 mg per eye per dose, but also can vary from this depending upon the activity of the agent and its solubility.

Thus, implants or microspheres can be prepared where the center may be of one material and the surface may have one or more layers of the same or a different composition, where the layers may be cross-linked, or of a different molecular weight, different density or porosity, or the like. For example, where it is desirable to quickly release an initial bolus of drug, the center of the microsphere may be a polylactate coated with a polylactate-polyglycolate copolymer, so as to enhance the rate of initial degradation. Alternatively, the center may be polyvinyl alcohol coated with polylactate, so that upon degradation of the polylactate exterior the center would dissolve and be rapidly washed out of the eye.

The implant or microspheres may be of any particulate geometry including micro and nanospheres, micro and nanoparticles, spheres, powders, fragments and the like. The upper limit for the microsphere size will be determined by factors such as toleration for the implant, size limitations on insertion, desired rate of release, ease of handling, etc. Spheres may be in the range of about 0.5 µm to 4 mm in diameter, with comparable volumes for other shaped particles.

The size and form of the implant or microspheres can also be used to control the rate of release, period of treatment, and drug concentration at the site of implantation. Larger microspheres will deliver a proportionately larger dose, but depending on the surface to mass ratio, may have a slower release rate. The particular size and geometry of the implant or microspheres are chosen to suit the activity of the active agent and the location of its target tissue.

The proportions of the valproic acid, polymer, and any other modifiers may be empirically determined by formulating several microsphere batches with varying average proportions. A USP approved method for dissolution or release test can be used to measure the rate of release (USP 23; NF 18 (1995) pp. 1790-1798). For example, using the infinite sink method, a weighed sample of the microspheres is added to a measured volume of a solution containing 0.9% NaCl in water, where the solution volume will be such that the drug concentration is after release is less than 5% of saturation. The mixture is maintained at 37°C and stirred slowly to maintain the microspheres in suspension. The appearance of the dissolved drug as a function of time may be followed by various methods known in the art, such as spectrophotometrically, HPLC, mass spectroscopy, etc. until the absorbance becomes constant or until greater than 90% of the drug has been released.

In addition to the valproic acid included in the implants and microspheres disclosed herein, the microsphere may also include one or more additional ophthalmically acceptable therapeutic agents. For example, the microspheres may include one or more antihistamines, one or more antibiotics, one or more beta blockers, one or more steroids, one or more antineoplastic agents, one or more immunosuppressive agents, one or more antiviral agents, one or more antioxidant agents, and mixtures thereof. Alternatively, a single injection of implant or microspheres can include two or more microsphere batches each containing a different therapeutic agent or agents. Such a mixture of different implants and microspheres in included within the present invention.

Additional pharmacologic or therapeutic agents which may find use in the present systems, include, without limitation, those disclosed in U.S. Pat. Nos. 4,474,451, columns 4-6 and 4,327,725, columns 7-8.

Examples of antihistamines therapeutic agents include, and are not limited to, loradatine, hydroxyzine, diphenhydramine, chlorpheniramine, brompheniramine, cyproheptadine, terfenadine, clemastine, triprolidine, carbinoxamine, diphenylpyraline, phenindamine, azatadine, tripelennamine, dexchlorpheniramine, dexbrompheniramine, methdilazine, and trimprazine doxylamine, pheniramine, pyrilamine, chiorcyclizine, thonzylamine, and derivatives thereof.

Examples of antibiotic therapeutic agents include without limitation, cefazolin, cephradine, cefaclor, cephapirin, ceftizoxime, cefoperazone, cefotetan, cefutoxime, cefotaxime, cefadroxil, ceftazidime, cephalexin, cephalothin,, cefamandole, cefoxitin, cefonicid, ceforanide, ceftriaxone, cefadroxil, cephradine, cefuroxime, ampicillin, amoxicillin, cyclacillin, ampicillin, penicillin G, penicillin V potassium, piperacillin, oxacillin, bacampicillin, cloxacillin, ticarcillin, azlocillin, carbenicillin, methicillin, nafcillin, erythromycin, tetracycline, doxycycline, minocycline, aztreonam, chloramphenicol, ciprofloxacin hydrochloride, clindamycin, metronidazole, gentamicin, lincomycin, tobramycin, vancomycin, polymyxin B sulfate, colistimethate, colistin, azithromycin, augmentin, sulfamethoxazole, trimethoprim, and derivatives thereof.

Examples of beta blocker therapeutic agents include acebutolol, atenolol, labetalol, metoprolol, propranolol, timolol, and derivatives thereof.

Examples of steroid therapeutic agents include corticosteroids, such as cortisone, prednisolone, flurometholone, dexamethasone, medrysone, loteprednol, fluazacort, hydrocortisone, prednisone, betamethasone, prednisone, methylprednisolone, riamcinolone hexacatonide, paramethasone acetate, diflorasone, fluocinonide, fluocinolone, triamcinolone, derivatives thereof, and mixtures thereof.

Examples of antineoplastic therapeutic agents include adriamycin, cyclophosphamide, actinomycin, bleomycin, duanorubicin, doxorubicin, epirubicin, mitomycin, methotrexate, fluorouracil, carboplatin, carmustine (BCNU), methyl-CCNU, cisplatin, etoposide, interferons, camptothecin and derivatives thereof, phenesterine, taxol and derivatives thereof, taxotere and derivatives thereof, vinblastine, vincristine, tamoxifen, etoposide, piposulfan, cyclophosphamide, and flutamide, and derivatives thereof.

Examples of immunosuppressive therapeutic agents include cyclosporine, azathioprine, tacrolimus, and derivatives thereof.

Examples of antiviral therapeutic agents include interferon gamma, zidovudine, amantadine hydrochloride, ribavirin, acyclovir, valciclovir, dideoxycytidine, phosphonoformic acid, ganciclovir, and derivatives thereof.

Examples of antioxidant therapeutic agents include ascorbate, alpha-tocopherol, mannitol, reduced glutathione, various carotenoids, cysteine, uric acid, taurine, tyrosine, superoxide dismutase, lutein, zeaxanthin, cryotpxanthin, astazanthin, lycopene, N-acetyl-cysteine, carnosine, gamma-glutamylcysteine, quercitin, lactoferrin, dihydrolipoic acid, citrate, Ginkgo Biloba extract, tea catechins, bilberry extract, vitamins E or esters of vitamin E, retinyl palmitate, and derivatives thereof.

Other therapeutic agents include squalamine, carbonic anhydrase inhibitors, alpha-2 adrenergic receptor agonists, antiparasitics, antifungals, and derivatives thereof.

The amount of therapeutic agent or agents employed in the implants and microspheres, individually or in combination, will vary widely depending on the effective dosage required and the desired rate of release from the microspheres. Usually the agent will be at least about 1, more usually at least about 10 weight percent of the microsphere, and usually not more than about 80, more usually not more than about 40 weight percent of the microspheres.

Some of the present implants and microspheres can comprise a combination of two or more different valproic acids or salts.

As discussed herein, the present implants and microspheres may comprise additional therapeutic agents. For example, one implant or microspheres dosage can comprise a combination of valproic acid and a beta-adrenergic receptor antagonist. More specifically, the microsphere or dosage of microspheres may comprise a combination of valproic acid and Timolol®. Or, a microsphere or dosage of microspheres may comprise a combination of valproic acid and a carbonic anyhdrase inhibitor. For example, the microsphere or dosage of microspheres may comprise a combination of valproic acid and dorzolamide (Trusopt®).

In addition to the therapeutic agent, the implants and microspheres disclosed herein may include or may be provided in drug delivery systems that include effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents advantageously present in amounts sufficient to maintain a pH of the system of between about 2 to about 9 and more preferably about 4 to about 8. As such the buffering agent may be as much as about 5% by weight of the total implant. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from about 0.001 % to about 5% by weight and preferably about 0.01 % to about 2% by weight. In at least one of the present microspheres, a benzylalkonium chloride preservative is provided in the implant.

In some situations mixtures of implants and microspheres may be utilized employing the same or different pharmacological agents. In this way, a cocktail of release profiles, giving a biphasic or triphasic release with a single administration is achieved, where the pattern of release may be greatly varied.

Additionally, release modulators such as those described in U. S. Patent No. 5,869,079 may be included in the implants or microspheres. The amount of release modulator employed will be dependent on the desired release profile, the activity of the modulator, and on the release profile of the valproic acid in the absence of modulator. Electrolytes such as sodium chloride and potassium chloride may also be included in the microspheres. Where the buffering agent or enhancer is hydrophilic, it may also act as a release accelerator. Hydrophilic additives act to increase the release rates through faster dissolution of the material surrounding the drug in the microspheres, which increases the surface area of the drug exposed, thereby increasing the rate of drug bioerosion. Similarly, a hydrophobic buffering agent or enhancer dissolves more slowly, slowing the exposure of drug, and thereby slowing the rate of drug bioerosion.

In certain microspheres, the combination of valproic acid and a biodegradable polymer matrix is released or delivered an amount of valproic acid between about 0.1 mg to about 0.5 mg for about 3-6 months after implantation or injection into the eye.

Various techniques may be employed to produce the implants and/or microspheres described herein. Useful techniques include, but are not necessarily limited to, self-emulsification methods, super critical fluid methods, solvent evaporation methods, phase separation methods, spray drying methods, grinding methods, interfacial methods, molding methods, injection molding methods, combinations thereof and the like.

As discussed herein, the polymeric component of the drug delivery system can comprise a biodegradable polymer or biodegradable copolymer. In at least one embodiment, the polymeric can comprise a poly (lactide-co-glycolide) PLGA copolymer. In a further embodiment, the PLGA copolymer has a lactide/glycolide ratio of 75/25. In a still further embodiment, the PLGA copolymer has at least one of a molecular weight of about 63 kilodaltons and an inherent viscosity of about 0.6 dL/g.

The present methods may also comprise a step of forming a first composition which comprises a valproic acid, a polymer, and an organic solvent, and a step of forming a second oil-containing composition, and mixing the first composition and the second oil-containing composition.

In addition, the present population of microparticles may have a maximum particle diameter less than about 200 µm. In certain embodiments, the population of microparticles has an average or mean particle diameter less than about 50 µm. In further embodiments, the population of microparticles has a mean particle diameter from about 30 µm to about 50 µm.

The present implants and microparticles are structured or configured to release the valproic acid for extended periods of time at controlled rates. In some embodiments, the valproic acid is released at a substantially linear rate (e.g., a single rate) over the life of the microparticles (e.g., until the microparticles fully degrade). Other embodiments are capable of releasing the valproic acid at multiple rates or different rates over the life of the microparticles. The rate at which the microparticles degrade can vary, as discussed herein, and therefore, the present microparticles can release the valproic acid for different periods of time depending on the particular configuration and materials of the microparticles. In at least one embodiment, a microparticle can release about 1 % of the valproic acid in the microparticles per day. In a further embodiment, the microparticles may have a release rate of about 0.7% per day when measured *in vitro.* Thus, over a period of about 40 days, about 30% of the valproic acid may have been released.

As discussed herein, the amount of the valproic acid present in the implants and microspheres can vary. In certain embodiments, about 10 to 30 wt% of the microspheres is the valproic acid. In further embodiments, the valproic acid constitutes about 20 wt% of the microspheres.

The microspheres, including the population of microspheres, of the present invention may be inserted into the subconjunctival (i.e. sub-tenon) space or into the vitreous of an eye by a variety of methods. The method of placement may influence the therapeutic agent or drug release kinetics. A preferred means of administration of the microspheres of the present invention is by subconjunctival injection. The location of the site of injection of the implants or microspheres may influence the concentration gradients of therapeutic agent surrounding the element, and thus influence the delivery rate to a given tissue of the eye. For example, an injection into the conjunctiva toward the posterior of the eye will direct drug more efficiently to the tissues of the posterior segment, while a site of injection closer to the anterior of the eye (but avoiding the cornea) may direct drug more efficiently to the anterior segment.

Microparticles may be administered to patients by administering an ophthalmically acceptable composition which comprises the microparticles to the patient. For example, microparticles may be provided in a liquid composition, a suspension, an emulsion, and the like, and administered by injection or implantation into the subconjunctival space of the eye.

The present implants or microparticles are configured to release an amount of valproic acid effective to treat an ocular condition (such as a retinal disease or condition) such as by reducing at least one symptom of the ocular condition. More specifically, the microparticles may be used in a method to treat. Additionally, subconjunctival or intravitreal delivery of microspheres containing valproic acid is able to provide quite high concentrations of the therapeutic agent to the retina of the eye.

The valproic acid containing implants and microspheres disclosed herein can also be configured to release the valproic acid with or without additional agents, as described above, which to prevent or treat diseases or conditions, such as the following: maculopathies/retinal degeneration: macular degeneration, including age related macular degeneration (ARMD), such as non-exudative age related macular degeneration and exudative age related macular degeneration, choroidal neovascularization, retinopathy, including diabetic retinopathy, acute and chronic macular neuroretinopathy, central serous chorioretinopathy, and macular edema, including cystoid macular edema, and diabetic macular edema. Uveitis/retinitis/choroiditis: acute multifocal placoid pigment epitheliopathy, Behcet's disease, birdshot retinochoroidopathy, infectious (syphilis, lyme, tuberculosis, toxoplasmosis), uveitis, including intermediate uveitis (pars planitis) and anterior uveitis, multifocal choroiditis, multiple evanescent white dot syndrome (MEWDS), ocular sarcoidosis, posterior scleritis, serpignous choroiditis, subretinal fibrosis, uveitis syndrome, and Vogt-Koyanagi-Harada syndrome. Vascular diseases/exudative diseases: retinal arterial occlusive disease, central retinal vein occlusion, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angitis, sickle cell retinopathy and other hemoglobinopathies, angioid streaks, familial exudative vitreoretinopathy, Eales disease. Traumatic/surgical: sympathetic ophthalmia, uveitic retinal disease, retinal detachment, trauma, laser, PDT, photocoagulation, hypoperfusion during surgery, radiation retinopathy, bone marrow transplant retinopathy. Proliferative disorders: proliferative vitreal retinopathy and epiretinal membranes, proliferative diabetic retinopathy. Infectious disorders: ocular histoplasmosis, ocular toxocariasis, presumed ocular histoplasmosis syndrome (POHS), endophthalmitis, toxoplasmosis, retinal diseases associated with HIV infection, choroidal disease associated with HIV infection, uveitic disease associated with HIV Infection, viral retinitis, acute retinal necrosis, progressive outer retinal necrosis, fungal retinal diseases, ocular syphilis, ocular tuberculosis, diffuse unilateral subacute neuroretinitis, and myiasis. Genetic disorders: retinitis pigmentosa, systemic disorders with associated retinal dystrophies, congenital stationary night blindness, cone dystrophies, Stargardt's disease and fundus flavimaculatus, Bests disease, pattern dystrophy of the retinal pigmented epithelium, X-linked retinoschisis, Sorsby's fundus dystrophy, benign concentric maculopathy, Bietti's crystalline dystrophy, pseudoxanthoma elasticum. Retinal tears/holes: retinal detachment, macular hole, giant retinal tear. Tumors: retinal disease associated with tumors, congenital hypertrophy of the RPE, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, intraocular lymphoid tumors. Miscellaneous: punctate inner choroidopathy, acute posterior multifocal placoid pigment epitheliopathy, myopic retinal degeneration, acute retinal pigment epithelitis and the like.

In one embodiment, a method of treating a retinal disease comprises administering a microsphere containing valproic acid, as disclosed herein, to a patient by subconjuctival injection. A syringe apparatus including an appropriately sized needle, for example, a 22 gauge needle, a 27 gauge needle or a 30 gauge needle, can be effectively used to inject the composition with into the subconjunctival space of an eye of a human or animal. Frequent repeat injections are often not necessary due to the extended release of the valproic acid from the microspheres.

In certain implants, the microspheres or implants consist essentially of valproic acid, salts thereof, and mixtures thereof, and a biodegradable polymer matrix. The biodegradable polymer matrix may consist essentially of PLA, PLGA, or a combination thereof. When placed in the eye, the preparation releases about 40% to about 60% of the valproic acid to provide a loading dose of the valproic acid within about one day after intraocular administration. Subsequently, the implant or microspheres release about 1 % to about 2% of the valproic acid per day to provide a sustained therapeutic effect.

Other microspheres disclosed herein may be configured such that the amount of the valproic acid that is released from the microspheres within two days of subconjunctival injection is less than about 95% of the total amount of the valproic acid in the microspheres. In certain formulations, 95% of the valproic acid is not released until after about one week of injection. In certain microsphere formulations, about 50% of the valproic acid is released within about one day of placement in the eye, and about 2% is released for about 1 month after being placed in the eye. In other microspheres, about 50% of the valproic acid is released within about one day of subconjunctival administration, and about 1 % is released for about 2 months after such administration.

A drug delivery system (such as an implant or microspheres) within the scope of our invention can be formulated with a high viscosity, polymeric gel to reduce dispersion of the composition upon intraocular injection. Preferably, the gel has a high shear characteristic, meaning that the gel can be injected into an intraocular site through a 25-30 gauge needle, and more preferably through a 27-30 gauge needle. A suitable gel for this purpose can be a hydrogel or a colloidal gel formed as a dispersion in water or other aqueous medium. Examples of suitable gels include synthetic polymers such as polyhydroxy ethyl methacrylate, and chemically or physically crosslinked polyvinyl alcohol, polyacrylamide, poly(N-vinyl pyrolidone), polyethylene oxide, and hydrolysed polyacrylonitrile. Examples of suitable hydrogels which are organic polymers include covalent or ionically crosslinked polysaccharide-based hydrogels such as the polyvalent metal salts of alginate, pectin, carboxymethyl cellulose, heparin, hyaluronate (i.e. polymeric hyaluronic acid) and hydrogels from chitin, chitosan, pullulan, gellan, xanthan and hydroxypropylmethylcellulose. Commercially available dermal fillers (such as Hylafrom®, Restylane®, Sculptura™ and Radiesse) can be used as the high viscosity gel in embodiments of our pharmaceutical composition.

Hyaluronic acid ("HA") is a polysaccharide made by various body tissues. U.S. patent 5,166,331 discusses purification of different fractions of hyaluronic acid for use as a substitute for intraocular fluids and as a topical ophthalmic drug carrier. Other U.S. patent applications which discuss ocular uses of hyaluronic acid include serial numbers 11/859,627; 11/952,927;10/966,764; 11/741,366; and 11/039,192 The pharmaceutical compositions within the scope of our invention preferably comprise a high viscosity hyaluronic acid with an average molecular weight between about 1 and 4 million Daltons, and more preferably with an average molecular weight between about 2 and 3 million Daltons, and most preferably with an average molecular weight of about (± 10%) 2 million Daltons.

Dry uncrosslinked HA material comprises fibers or powder of commercially available HA, for example, fibers or powder of sodium hyaluronate (NaHA). The HA may be bacterial-sourced sodium hyaluronate, animal derived sodium hyaluronate or a combination thereof. In some embodiments, the dry HA material is a combination of raw materials including HA and at least one other polysaccharide, for example, glycosaminoglycan (GAG).

In our invention the HA used comprises or consists of high molecular weight HA. That is, nearly 100% of the HA material in the present compositions is a high molecular weight HA. High molecular weight HA means HA with a molecular weight of at least about 1.0 million Daltons (mw ≥ 10⁶ Da) to about 4.0 million Da (mw ≤ 4 X 10⁶ Da). For example, the high molecular weight HA in the present compositions may have a molecular weight of about 2.0 million Da (mw 2 X 10⁶ Da). In another example, the high molecular weight HA may have a molecular weight of about 2.8 million Da (mw 2.8 x 10⁶ Da).

In an embodiment of our invention, dry or raw HA material (in this specific example, NaHA) having a desired high/low molecular weight ratio is cleaned and purified. These steps generally involved hydrating the dry HA fibers or powder in the desired high/low molecular weight ratio, for example, using pure water, and filtering the material to remove large foreign matters and/or other impurities. The filtered, hydrated material is then dried and purified. The high and low molecular weight NaHA may be cleaned and purified separately, or may be mixed together, for example, in the desired ratio, just prior to crosslinking.

At this stage in the process, the pure, dried NaHA fibers are hydrated in an alkaline solution to produce an uncrosslinked NaHA alkaline gel. Any suitable alkaline solution may be used to hydrate the NaHA in this step, for example, but not limited to an aqueous solution containing NaOH. The resulting alkaline gel will have a pH above 7.5, for example, a pH above 8, for example, a pH above 9, for example, a pH above 10, for example, a pH above 12, for example, a pH above 13.

In this specific example, the next step in the manufacturing process comprises the step of crosslinking the hydrated, alkaline NaHA gel with a suitable crosslinking agent, for example, BDDE.

The step of HA crosslinking may be carried out using means known to those of skill in the art. Those skilled in the art appreciate how to optimize the conditions of crosslinking according to the nature of the HA, and how to carry out the crosslinking to an optimized degree.

In some embodiments of the present invention, the degree of crosslinking is at least about 2% to about 20%, for example, is about 4% to about 12%, wherein the degree of crosslinking is defined as the percent weight ratio of the crosslinking agent to HA-monomeric units in the composition.

The hydrated crosslinked, HA gel may be neutralized by adding an aqueous solution containing HCl. The gel is then swelled in a phosphate buffered saline solution for a sufficient time and at a low temperature.

In certain embodiments, the resulting swollen gel (HA) is a cohesive gel having substantially no visible distinct particles, for example, substantially no visibly distinct particles when viewed with the naked eye. In some embodiments, the gel has substantially no visibly distinct particles under a magnification of less than 35X.

The gel ((HA) is now purified by conventional means for example, dialysis or alcohol precipitation, to recover the crosslinked material, to stabilize the pH of the material and remove any unreacted crosslinking agent. Additional water or slightly alkaline aqueous solution can be added to bring the concentration of the NaHA in the composition to a desired concentration. In some embodiments, the concentration of NaHA in the composition is in a range between about 10 mg/ml to about 30 mg/ml.

### EXAMPLES

### Example 1

### In Vitro Identification of Valproic Acid for Treating Retinal Cell Oxidative Stress

In this experiment we obtained evidence that valproic acid can protect retinal cells from oxidative insult (oxidative stress). Thus we examined changes in gene expression of cultured retinal cells as a result of sub-lethal oxidative stress under acute and chronic treatment conditions and in both the acute and chronic treatment samples determined using a connectivity map that valproic acid has potential to mediate the observed genes. The connectivity map used (based at The Broad Institute of MIT and Harvard in Cambridge, Massachusetts and available online at www.broad.mit.edu/cmap) is a collection of genome-wide transcriptional expression data from cultured human cells treated with bioactive small molecules and pattern-matching algorithms.

The experiment was carried out as follows. ARPE-19 cells were grown to confluence at 37°C., in 0.5% CO₂, in a humidified incubator and after reaching confluence individual cultures were treated for 1 hr. with increasing dosage of tert-butyl hydroperoxide (tBH), 60, 300 and 600 um at 37°C., 0.5% CO₂, in a humidified incubator. After the 1 hr. period fresh culture media was added and cultures returned to 37°C., 0.5% CO₂, in a humidified incubator. This treatment was carried out once (24 hr data set) or four times every 24 hrs. over a 4 day period resulting in 4 treatments/tBH concentration (94 hr data set). 24 hrs after the last treatment cells were harvested and mRNA extracted. Isolated mRNA was then used to probe Affymetrix Hu133 Plus 2.0 microarray using standard conditions. This process was repeated thrice generating three independent experiments and data sets for both the single and repeated treatment protocols. The three independent experiments were then analyzed by normalizing the data across the three experiments using principle component analysis and then running ANOVA on the normalized data using p=0.001 to identify transcripts showing a significant change in regulation associated with tBH treatment. Based on the resulting gene data set, they were further filtered to select genes only showing a tBH dose dependent change in expression levels. The resulting data set was then separated into up and down regulated genes and the two gene lists used to construct a connectivity map and identified valproic acid as inducing similar or opposite changes in the gene sets as that observed with tBH treatment in ARPE-19 cells. From this gene array analysis we identified valproic acid as having utility for treating oxidative stressed retinal cells.

In this experiment we carried out an *in vitro* microarray retinal cell chronic oxidative stress study and determined that valproic acid may regulate gene changes induced by oxidative stress thereby showing potential use of valproic acid as a therapeutic agent for protecting ocular tissues from oxidative insult associated with retinal diseases, such as AMD (age related macular degeneration, diabetic retiniopathy, retinal ranch vein occlusion and glaucoma.

### Example 2

### In Vivo Use of Valproic Acid to Treat Oxidative Stressed Retina

In furtherance of the *in vitro* results of Example 1 we carried out an *in vivo* experiment examining the ability of valproic acid to protect against oxidative insult (oxidative stress) to mammalian retina. We used a murine model in which oxidative stress is induced by pre-dosing with intravitreal paraquat in Sod1^{tm1Leb/J} mouse model (as set forth in Dong A., et al., Superoxide Dismutase 1 Protects Retinal Cells From Oxidative Damage, J Cell Physio 208:516-526, 2006).

C57BL6 mice heterozygous from the SOD1 gene were obtained from Jackson labs (strain B6;129S7-Sod1tm1Leb/J). The mice were 3- 4 months of age at the time of the experiment. Mice were divided into two groups (4 in each group) both groups receiving paraquat injections in the OD (right) eye while the OS (left) eye was untreated and used as control for electroretinogram (ERG) measurements. The treatment group received valproic acid intraperitoneally (IP) while the control group was untreated.

Valproic acid was prepared at a concentration of 62.5 mg/mL in sterile phosphate buffered saline (PBS). Beginning three days prior to the initiation of paraquat model and throughout the experimental period 250 mg/kg valproic acid was given by IP injection once daily.

The mice were anesthetized with ketamine (100 mg/kg) plus xylazine (50 mg/kg), IP and kept on a heating pad. Both eyes were kept moist with 1-2 drops of Celluvisc. A 36 gauge needle attached to a Hamilton Lab animal injector syringe (LASI 115) was inserted distal to the ora serrata, penetrating into the vitreous humor, avoiding disruption of the retinal and of the lens. A 1 ul injection of 0.75 mM paraquat was injected into the vitreous eye and the needle withdrawn.

ERG recording were measured on dark adapted animals on days 1 and 7 post paraquat injection using an Espion ERG Diagnosys system and Burian-Allen electrodes, 3.0 mm diameter from LKC Technologies, Inc.. For ERG recordings mice were anesthetized with ketamine (100 mg/kg) plus xylazine (50 mg/kg) administered IP and pupils dilated with 1% Akpentolate (cyclopentolate hydrochloride) and 10% AK-Dilate (phenylephrine hydrochloride). Celluvisc was then placed on the eyes and surface electrodes attached. Scotopic ERGs were recorded using a .001, .01, and 1 cd.s/m2 flash as well as a 20 Hz flicker with the animal on a heating pad. A-wave, B-wave amplitudes were then calculated and statistically significant changes between valproic treated and non-treated animals determined using T test, n=4.

From this *in vivo* experiment we determined that the mice administered 250 mg/kg valproic acid intraperitonally once a day beginning three days prior to initiation of oxidative insult and throughout the period of the experiment, had significantly inhibited paraquat induced decrease in B-wave amplitude (see Figure 1). Thus, Figure 1 shows a protective effects of valproic acid on inhibition of wave deficient in mice as a result of oxidative stress induced by intravitreal injection of paraquat in the Sod1^{tm1Leb/J} mice. I.V.T. Note that injection of Paraquat (0.75 mM) induced a decrease in the B-wave amplitude in the Sod1^{tm1Leb/J} mice (control vs paraquat) and that treatment 250 mg/Kg valproic acid suppressed paraquat induced deficient in B-wave amplitude (paraquat + valproic vs paraquat). These results showed that valproic acid provided protection to the neural retina against oxidative stress thereby pointing to utility of valproic acid as a therapeutic for treatment of retinal diseases.

This experiment identified valproic acid as having utility as a therapeutic agent for preventing damage due to oxidative stress in the retina. This is an unpredictable finding because it has been reported that valproic acid is associated with the generation of reactive oxygen species. Kawai, Y., et al., Valproic acid-induced gene expression through production of reactive oxygen species, Cancer Res. 6613: 6563-9 (2006).

### Example 3

### Methods and compositions for the intraocular delivery of valproic acid and valproate salts for the treatment of intraocular diseases

In this experiment we overcame the difficulties existing to formulate a valproic acid containing sustained release drug delivery system due to the fact that valproic acid is liquid at body temperature and that the valproic acid salt sodium valproate is highly water soluble and therefore also difficult to administer in a sustained release form. We developed systems and methods by which valproic acid or salts of valproic acid can be delivered in a sustained release drug delivery system. In one formulation, liquid valproic acid is combined with biodegradable polymers such as PLGA or PLA and/or other biocompatible pharmacologically safe compounds such as a polysaccharide or poly amino acid. The dry formulation is blended so that the valproic acid sorbs onto the surface and into the pores of the polymers and/or excipients. Valproic acid can comprise 1 to 50% of the total formulation, by weight. The powder blend is then formed into a drug delivery system either by hot melt extrusion or by direct compaction. In a second formulation, sodium valproate or another salt of valproic acid is combined with a biodegradable polymer such as PLGA or PLA as a dry powder blend. Other hydrophobic biologically inert excipients can be added to the powder blend to inhibit hydroscopicity. The powder blend is then processed into a drug delivery system either by piston extrusion, hot melt extrusion, or solvent casting. Dosage forms would be determined by total weight of extruded filament or cut film. Valproic acid salt can comprise 1 to 60% of the total weight of the drug delivery system and extrusion temperatures could range from 40°C to 180° C. In a third formulation, valproic acid salt is co-solubilized in water with a cationic polyelectrolyte such as chitosan. The cationic polyelectrolyte complexes with the disassociated sodium carboxylate group on the sodium valproate. The solution is then lyophilized to a dry powder. The lyophilized powder is combined with a biodegradable polymer using dry powder blending techniques. The formulation is then extruded using piston or twin-screw hot melt extrusion. The unit dosage is determined by sodium valproate to polyelectrolyte ratio, percent lyophilized powder incorporated into the formulation by weight, and by total filament weight post-extrusion.

A particular valproic acid implant can be made as follows. A 20 wt % valproic acid containing bioerodible polymer (80 wt % polymer; RG752s and/or R202s) implant can be made by hot-melt extrusion using a mechanically driven ram microextruder but can also be made by direct compression or solvent casting. The implants can be rod-shaped, but they can be made into any geometric shape by changing the extrusion or compression die. The valproic acid and the polymer are initially mixed using a spatula in a weigh-boat for 15 minutes. The samples are then transferred into a stainless steel container containing two ¼" stainless steel ball and mixing continued using a Turbula mixer for two separate 15 minute cycles. The powder blend is mixed by hand using a spatula between each cycle and after the final cycle. The blended material is compacted into an extruder barrel and the extruder barrel is placed into the heated well (between 80 and 120 degrees C.) of the piston extruder and extruded using 500 pm nozzle and a speed setting number of 0.0025. The RG752s polymer resomer has an inherent viscosity of from 0.16 to .024 dl/g and R202s resomer has an inherent viscosity of 0.2 dl/g and these resomers have average molecular weights of about 11,200 and 6,500, respectively. The extruded filaments are cut into one milligram implant (approximately 3 mm long), and (for *in vitro* release study) placed into a 10 ml vial containing 0.01 M phosphate buffered saline (pH 7.4), and then transferred into a shaking water bath set at 37°C and 50 rpm. At various time points, the solution is removed and analyzed by HPLC to determine the amount of Valproic acid released by the implants. The removed solution is replaced with fresh phosphate buffered saline solution until a release profile is determined.

### Example 4

### Method for Making Valproic Acid Microspheres

Valproic acid containing microspheres can be made by dissolving 20 mg of valproic acid and 100 mg polymer (Resomer 203H) in 0.8 ml ethyl acetate. A minimum amount of dichloromethane is added to complete dissolution. Then added to this solution is 40 ml 1 % polyvinyl acetate in water via a micro-pipette while shearing the mixture at 3000 rpm for 5 minutes with a Silverson homogenizer. When the polymer solution is added to the water, the pipette tip is submerged under the water surface and added dropwise.

After shearing, a milky white emulsion is formed and it is mildly agitated in a hood for 3-5 hrs to allow solvent evaporation. This suspension is filtered through a 106 um sieves, and particle size is measured. The suspension is then centrifuged at 2000 rpm for 15 min to remove supernatant, followed by adding 10 mL distilled water to reconstitute the microspheres. Finally the microspheres are lyophilized followed by drug content assay, and *in vitro* release assay. Typical microsphere diameters are about 35 um, with 13% valproic acid loading.

Samples of the microspheres were formulated with a high viscosity hyaluronic acid. Thus, 10 mg microspheres are mixed with 100 uL Captique gel and another 10 mg microsphere sample was mixed with 50 uL J18 gel. Instead of Captique gel Juvederm Ultra Plus or Voluma (both available from Allergan, Irvine, California) can be used instead.

A number of batches of valproic acid microspheres can be made using various known bioerodible polymers, such as R203H and RG502H. First day *in vitro* release (in PBS medium with 0.1% triton 100) rates can vary from 3% to 60% of the valproic acid, and microsphere mean diameter being between 18 and 52 microns.

### Example 5

### Treatment of Neovascularization

A 68 year old woman complains of blurry vision in her left eye and is seen by her general ophthalmologist. She has visual acuity of CF 3 ft left eye with an ischemic central retinal vein occlusion with numerous cotton wool spots apparent in the posterior pole. The patient is watched closely and develops macula neovascularization 3 months following the vein occlusion. The intraocular pressure (IOP) increases to 42 mmHg and the angle can show fine new vessels coursing through the retina, trebecular meshwork with anterior synechiae noted temporally. The patient can receive a subTenon's or intravitreal injection of an Example 3 or 4 valproic acid drug delivery system. After 2 weeks, the IOP can be 26 mmHg both the iris and retinal neovascularization neovascularization improved.

### Example 6

### Treatment of Macular Degeneration

A 76 year old man has age-related macular degeneration and cataracts in both eyes. The patient can also have a history of cardiovascular disease and an inferior wall myocardial infarction 6 months previous. The patient can complain of blurry vision and metamorphopsia in the right eye and examination can reveal visual acuity of 20/400 right eye, 20/32 left eye. Retinal examination can show subfoveal choroidal neovascularization (CNV) (right eye wet AMD) approximately 1 disc area in size with surrounding hemorrhage and edema in the right eye. The fellow left eye can show high-risk features for developing wet AMD such as soft, amorphic appearing drusen that included the fovea but no signs of choroidal neovascularization and can be confirmed by fluorescein angiography (left eye dry AMD)

In both eyes the patient can receive an intravitreal injection of a valproic acid drug delivery made according to Example 3 or 4. The injected volume can be 50 ul comprising valproic acid incorporated into PLGA microspheres with a total valproic acid weight of 2.5 mg.

The patient can receive the intravitreal left eye injections of the 50 ul of valproic acid-PLGA microspheres (total drug weight 2.5 mg) invention every 6 months and at the end of a 7-year follow up period the patient can have maintained vision in the both eyes of at least 20/32.

## Claims

1. An intraocular drug delivery system for use in treating a retinal condition, comprising:
(a) a bioerodible polymer, and;
(b) a valproic acid associated with the bioerodible polymer,
wherein the polymer is a polylactic polyglycolic acid copolymer (PLGA), a polylactic acid polymer (PLA) or a high viscosity hyaluronic acid.

2. The drug delivery system for use according to claim 1 wherein the polymer and/or the valproic acid comprises from about 10% to about 95% by weight of the drug delivery system.

3. The drug delivery system for use according to claim 1 wherein the valproic acid is associated with the polymer by being homogenous mixed with the polymer.

4. The drug delivery system for use according to claim 1, wherein the drug delivery system is an implant or a population of microspheres.

5. The drug delivery system claim 1, wherein the drug delivery system comprises a solution of the valproic acid in a high viscosity, polymeric hyaluronic acid.

6. An intraocular drug delivery implant for use in treating a retinal condition comprising 10 to 30 weight percent sodium valproate and 70 to 90 weight PLGA, the implant obtainable by homogenously mixing the valproate and the PLGA, then heating the mixture to a temperature between about 40 and about 180 C, followed by extrusion of the implant.

7. The intraocular drug delivery system for use according to claim 1, wherein the drug delivery system is administered to the vitreous or an intrascleral location.

8. The drug delivery system according to claim 7, wherein the drug delivery system is administered to a subtenon location.

9. The intraocular drug delivery system according to claim 1, wherein the drug delivery system is a sustained release monolithic implant capable of releasing a therapeutic amount of the valproic acid for between one week and one year.

10. The intraocular drug delivery system according to claim 1 for use in treating a macular edema, comprising the step of intravitreal administration to a patient with macula edema.

11. The drug delivery system according to claim 1 for use in reducing retinal tissue oxidative stress in a human patient, comprising the step of intravitreal administration to a human patient with oxidative stress retinal cells

12. The drug delivery system for use according to claim 11 , wherein the oxidative stress retinal tissue contains an excess level of a reactive oxygen species selected from the group consisting of peroxinitrate, super oxide, singlet oxygen, hydrogen peroxide, hypochlorite and hydroxy radical.

13. The drug delivery system for use according to claim 12 wherein the method reduces the excess level of reactive oxygen species to a normal level of reactive oxygen species as determined by a process selected from the group consisting of reactive oxygen sensing dyes, high-performance liquid chromatography (HPLC), immunohistochemistry, Western blotting, enzyme-linked-immunosorbent serologic assay (ELISA), tandem mass spectrometry (MS-MS) and presence or upregulation of oxidative stress response gene/protein or transcription factors.

## Patentansprüche

1. Intraokulares Arzneimittelabgabesystem zur Verwendung in der Behandlung einer Erkrankung der Retina, umfassend:
(a) ein biologisch abbaubares Polymer und;
(b) eine mit dem biologisch abbaubaren Polymer assoziierte Valproinsäure,
wobei das Polymer ein Polylactid-co-Glycolid (PLGA), ein Polylactid (PLA) oder eine hochviskose Hyaluronsäure ist.

2. Arzneimittelabgabesystem zur Verwendung nach Anspruch 1, wobei das Polymer und/oder die Valproinsäure etwa 10 Gewichts-% bis etwa 95 Gewichts-% des Arzneimittelabgabesystems ausmacht.

3. Arzneimittelabgabesystem zur Verwendung nach Anspruch 1, wobei die Valproinsäure mit dem Polymer durch homogenes Mischen mit dem Polymer assoziiert ist.

4. Arzneimittelabgabesystem zur Verwendung nach Anspruch 1, wobei das Arzneimittelabgabesystem ein Implantat oder eine Anzahl von Mikrosphären ist.

5. Arzneimittelabgabesystem zur Verwendung nach Anspruch 1, wobei das Arzneimittelabgabesystem eine Lösung der Valproinsäure in einer hochviskosen, polymeren Hyaluronsäure umfasst.

6. Intraokulares Arzneimittelabgabeimplantat zur Verwendung in der Behandlung einer Erkrankung der Retina, umfassend 10 bis 30 Gewichts-% Natriumvalproat und 70 bis 90 Gewicht PLGA, wobei das Implantat erhältlich ist durch homogenes Mischen des Valproats und des PLGA, dann Erhitzen der Mischung auf eine Temperatur zwischen etwa 40 und etwa 180°C, gefolgt von Extrusion des Implantats.

7. Intraokulares Arzneimittelabgabesystem zur Verwendung nach Anspruch 1, wobei das Arzneimittelabgabesystem an den Glaskörper oder einen intraskleralen Ort verabreicht wird.

8. Intraokulares Arzneimittelabgabesystem zur Verwendung nach Anspruch 7, wobei das Arzneimittelabgabesystem an einen subtenonen Ort verabreicht wird.

9. Intraokulares Arzneimittelabgabesystem zur Verwendung nach Anspruch 1, wobei das Arzneimittelabgabesystem ein monolithisches Depot-Implantat ist, das in der Lage ist, eine therapeutische Menge Valproinsäure für zwischen einer Woche und einem Jahr freizusetzen.

10. Intraokulares Arzneimittelabgabesystem nach Anspruch 1 zur Verwendung in der Behandlung eines Makulaödems, umfassend den Schritt der intravitrealen Verabreichung an einen Patienten mit Makulaödem.

11. Intraokulares Arzneimittelabgabesystem nach Anspruch 1 zur Verwendung in der Verringerung von oxidativem Stress von Retinagewebe in einem Menschen, umfassend den Schritt der intravitrealen Verabreichung an einen Menschen mit Retinazellen mit oxidativem Stress.

12. Intraokulares Arzneimittelabgabesystem zur Verwendung nach Anspruch 11, wobei das Retinagewebe mit oxidativem Stress einen Überschuss einer reaktiven Sauerstoffspezies, ausgewählt aus der Gruppe, die aus Peroxynitrat, Superoxid, Singulett-Sauerstoff, Wasserstoffperoxid, Hypochlorit und Hydroxylradikal besteht, enthält.

13. Intraokulares Arzneimittelabgabesystem zur Verwendung nach Anspruch 12, wobei das Verfahren den Überschuss der reaktiven Sauerstoffspezies auf einen normalen Gehalt der reaktiven Sauerstoffspezies reduziert, bestimmt durch ein Verfahren, das ausgewählt ist aus der Gruppe, die aus Sensorfarbstoffen für reaktiven Sauerstoff, Hochleistungsflüssigkeitschromatographie (HPLC), Immunohistochemie, Western Blotting, enzymgekoppeltem serologischen Immunadsorptionstest (ELISA), Tandem-Massenspektroskopie (MS-MS) und Anwesenheit oder Hochregulation von Antwortgen/Protein oder Transkriptionsfaktoren für oxidativen Stress besteht.

## Revendications

1. Système intraoculaire d'administration de médicament destiné à être utilisé dans le traitement d'un trouble rétinien, qui comprend :
(a) un polymère bioérodable, et ;
(b) un acide valproïque associé au polymère bioérodable, dans lequel le polymère est un copolymère d'acide polylactique-polyglycolique (PLGA), un polymère d'acide polylactique (PLA) ou un acide hyaluronique de haute viscosité.

2. Système d'administration de médicament destiné à être utilisé selon la revendication 1, dans lequel le polymère et/ou l'acide valproïque représentent d'environ 10 % à environ 95 % en poids du système d'administration de médicament.

3. Système d'administration de médicament destiné à être utilisé selon la revendication 1, dans lequel l'acide valproïque est associé au polymère par mélange homogène avec le polymère.

4. Système d'administration de médicament destiné à être utilisé selon la revendication 1, dans lequel le système d'administration de médicament est un implant ou une population de microsphères.

5. Système d'administration de médicament destiné à être utilisé selon la revendication 1, dans lequel le système d'administration de médicament comprend une solution de l'acide valproïque dans un acide hyaluronique polymère de haute viscosité.

6. Implant intraoculaire pour l'administration de médicament destiné à être utilisé dans le traitement d'un trouble rétinien qui comprend de 10 à 30 % en poids de valproate de sodium et de 70 à 90 % en poids de PLGA, l'implant pouvant être obtenu par mélange homogène du valproate et du PLGA, puis par chauffage du mélange à une température comprise entre environ 40 et environ 180°C, suivi par l'extrusion de l'implant.

7. Système intraoculaire d'administration de médicament destiné à être utilisé selon la revendication 1, dans lequel le système d'administration de médicament intraoculaire est administré au corps vitré ou à un emplacement intrascléral.

8. Système intraoculaire d'administration de médicament destiné à être utilisé selon la revendication 7, dans lequel le système d'administration de médicament est administré à un emplacement sous-épiscléral.

9. Système intraoculaire d'administration de médicament destiné à être utilisé selon la revendication 1, dans lequel le système d'administration de médicament est un implant monolithique à libération prolongée, capable de libérer une quantité thérapeutique de l'acide valproïque pendant une semaine à une année.

10. Système intraoculaire d'administration de médicament selon la revendication 1, destiné à être utilisé dans le traitement d'un oedème maculaire, qui comprend l'étape d'administration intravitréenne à un patient souffrant d'un oedème maculaire.

11. Système intraoculaire d'administration de médicament selon la revendication 1, destiné à être utilisé pour réduire le stress oxydatif du tissu rétinien chez un patient humain, qui comprend l'étape d'administration intravitréenne à un patient humain dont les cellules rétiniennes sont en état de stress oxydatif.

12. Système intraoculaire d'administration de médicament destiné à être utilisé selon la revendication 11, dans lequel le tissu rétinien en état de stress oxydatif contient un niveau excessif d'une espèce réactive de l'oxygène choisie dans le groupe constitué du péroxynitrite, d'un superoxyde, de l'oxygène singulet, du peroxyde d'hydrogène, de l'hypochlorite et d'un radical hydroxy.

13. Système intraoculaire d'administration de médicament destiné à être utilisé selon la revendication 12, dans lequel le procédé réduit le niveau excessif de l'espèce réactive de l'oxygène à un niveau normal de l'espèce réactive de l'oxygène tel que déterminé par un procédé choisi dans le groupe constitué des colorants de détection de l'oxygène réactif, de la chromatographie en phase liquide haute performance (CLHP), de l'immunohistochimie, d'un transfert de type Western, d'un ELISA (dosage immunoenzymatique), de la spectrométrie de masse en tandem (SM-SM) et de la présence ou de la régulation à la hausse d'un gène/une protéine ou de facteurs de transcription sensibles au stress oxydatif.
